Europäisches Patentamt

European Patent Office

Office européen des brevets

(19)

(11) Publication number: **0 039 715**
**B1**

(12) **EUROPEAN PATENT SPECIFICATION**

(45) Date of publication of patent specification: **14.09.83**

(51) Int. Cl.³: **A 41 B 13/02, A 61 F 13/18**

(21) Application number: **80902227.0**

(22) Date of filing: **11.11.80**

(86) International application number:
**PCT/FI80/00007**

(87) International publication number:
**WO 81/01358 14.05.81 Gazette 81/12**

(54) **BABY'S DIAPER OR EQUIVALENT.**

(30) Priority: **13.11.79 FI 793558**

(43) Date of publication of application:
**18.11.81 Bulletin 81/46**

(45) Publication of the grant of the patent:
**14.09.83 Bulletin 83/37**

(84) Designated Contracting States:
**DE FR GB SE**

(56) References cited:
**CH - A - 273 536**
**DE - A - 2 734 783**
**DE - B - 164 020**
**FR - A - 1 260 951**
**FR - A - 2 423 171**
**GB - A - 28 756**
**SE - C - 222 362**
**US - A - 1 169 490**
**US - A - 2 548 341**
**US - A - 2 600 882**
**US - A - 3 105 491**
**US - A - 3 523 536**

(73) Proprietor: **NUORTIE, Raimo**
**Kivenlahdenkatu 3 E 52**
**SF-02320 Espoo 32 (FI)**

(72) Inventor: **NUORTIE, Raimo**
**Kivenlahdenkatu 3 E 52**
**SF-02320 Espoo 32 (FI)**

(74) Representative: **Tiedtke, Harro, Dipl.-Ing. et al,**
**Patentanwaltsbüro Tiedtke-Bühling-Kinne- Grupe-**
**Pellmann-Grams Bavariaring 4**
**D-8000 München 2 (DE)**

(56) References cited:
**US - A - 3 612 054**
**US - A - 3 663 348**
**US - A - 3 777 758**

Courier Press, Leamington Spa, England

### Baby's diaper or equivalent

The present invention concerns a baby's diaper or equivalent, such as ladies' sanitary towel, consisting of a filler material containing free chemically manufactured cellulose fibres (chemical cellulose) or equivalent fibres, and comminuted newsprint or equivalent paper enclosed within a skin layer.

Another product equivalent to baby diapers and ladies' sanitary towels is the so-called chronic invalid's diaper, which also belongs within the scope of the present invention.

As known in prior art, baby diapers or equivalent consist of a filler material which is in the manufacturing process deposited upon tissue paper, and pieces are cut from the combination web thus obtained. The said pieces are folded triple, and such a combination of tissue web and filler material is enclosed in a bag made of non-woven web material.

As filler material in baby diapers of prior art is used cellulose and mechanical pulp, which has been comminuted e.g. in a hammer mill from either sheet cellulose or groundwood pulp.

Through the Finnish patent application No. 1537/70 is known in prior art a sanitary towel or diaper containing in addition to fibrous cellulose pulp, a substance which is elastic in wet as well as dry condition. In the said application has been specified as said resilient substance, e.g. granular foamed plastic.

The U.S. Patent No. 2,548,341 discloses a towel wherein as filler material cellucotton is used and paper, ground very fine and to be lint-like. Since according to this reference the paper has been ground very fine and to be lint-like, in other words into single fibres, the effects aimed at in the present invention are not achieved thereby.

It is a drawback of diapers and equivalent of prior art and of their filler materials that when cellulose fibres are used for filler material the diaper cannot be made bulky (fluffy) enough, nor is any high enough resilience and permanence of shape achieved. It has further been a drawback that even though the diaper as a whole may be absorbent enough, local leakages can occur because the user's excretions do not nearly spread uniformly into the whole volume of the diaper, instead of which they are concentrated on a small area. Also that may be considered detrimental that fibrous cellulose in itself is a comparatively expensive material, while on the other hand cellulose has rather good absorption properties. The above-mentioned drawbacks are partly due to the fact that in the manufacturing process the separate cellulose fibres are oriented in a given direction and hereby the bulkiness of the product is impaired and its elasticity reduced because a fibre network with substantially uniform orientation in every direction fails to form wherein bending of the fibres would give rise to elasticity and to permanence of shape and to bulk.

With a view to eliminating the drawbacks mentioned, the invention is mainly characterized in that one uses, in addition to fibrous cellulose or equivalent, for filler material: fine-chopped newsprint and/or other equivalent paper, the paper being substantially in particles having a largest dimension of 3—10 mm and the proportion of paper in the filler material being 40—60% by weight such that the diaper's elasticity and/or bulk, absorbence and liquid spread-out are substantially improved.

If the paper component were ground into completely single fibres, it would then conform to fibrous cellulose, and by means of such a component one would not be able to increase the diaper's elasticity and/or bulk, absorbence and liquid spread-out over the whole diaper volume, which advantages are specifically implemented when there is used as filler material, newsprint chopped into appropriate particles as taught by the invention.

It is a substantial advantage afforded by the diaper of the invention that newsprint particles brings about the result that liquid will spread out uniformly in the diaper owing to capillary phenomena, so that the diaper is wetted all the way up to its ends, and whereby the full absorbing capacity of the diaper is utilized. It has been found in practice that the diaper of the invention when it becomes wet acquires a stratified structure so that local penetration of liquids is inhibited and so that even when wet the diaper possesses in all directions sufficient strength, with the result of reduced breaking of the diaper in wet condition.

Also another advantage of the invention is obvious from the foregoing: that as generally the wetting effect acts on the diaper quite locally, the diaper will not begin to run at this point because thanks to the newsprint particles liquids spread out more efficiently than before from one end to the other of the diaper.

The invention is described in detail in the following, with reference being made to a certain embodiment example of the invention, presented in the figure of the attached drawing.

Fig. 1 displays the cross section of a diaper of the invention in substantially natural size.

Fig. 2 shows a partial section, paralleling the plane of the diaper, of the diaper filling.

Fig. 3 illustrates an apparatus by which diapers according to the invention are manufactured.

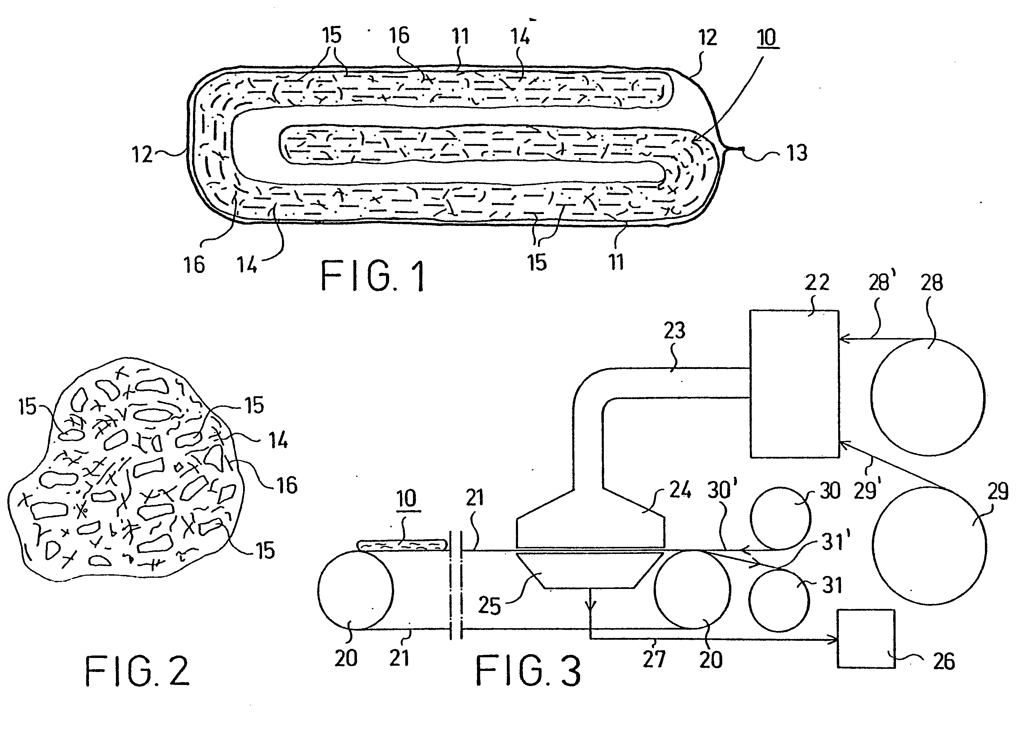

Referring now to Figs. 1 and 2, the diaper 10 therein depicted, or equivalent such as a ladies' sanitary towel for instance, consists of a filler material 14, which is by means of an apparatus such as Fig. 3 shows, when manufacturing diapers 10, conveyed onto a tissue web 11. From the combination web 11, 14 produced in the manner described, lengths are cut as known in itself in the art and these are folded to become a tri-ply part as shown in Fig. 1, and such an entity is enclosed in a bag consisting of a non-woven web 12, which is provided with a seam 13.

It is substantially novel in the invention that the filler material 14 of the diaper is not made in its entirety of loose cellulose fibres, but instead as material for the filler material 14 is used newsprint or other equivalent paper or cardboard, which has been ground into suitable particles before its introduction in the filler material. According to a favourable embodiment of the invention, one uses in the filler material 14, between 40 and 60% by weight of newsprint, the other filler material component consisting of free, chemically manufactured cellulose fibres. Excellent preliminary experiences were gained with a diaper containing 50% newsprint and 50% cellulose, the newsprint having been comminuted in a hammer mill 22 (Fig. 3) partly to particles 15 and partly to fibres 16, which are rather long, above all when newsprint is used which has been made partially of mechanical groundwood, for instance of thermogroundwood. For the other component of the diaper filler material one will use to advantage such newsprint or equivalent which has a basis weight between 30 and 80 g per m². It is furthermore advantageous to use for said other component a paper web containing no fillers, or not much of them at least.

When newsprint is being turned out on a paper machine, rather large quantities of such paper are produced which is lacking in quality for newspaper printing, but which is eminently suitable to be used to the purposes of the present invention.

When newsprint or equivalent is being used for filler material of diapers as taught by the invention, the characteristics of the diapers improve, for the following reasons. Particularly newsprint made partly from thermogroundwood contains long fibres. These long fibres are partly separated in the grinding process in the newsprint and hammer mill, and the fibres increase the elasticity and bulk of the product. When the newsprint or equivalent is ground in such manner that newsprint snips are left in the filler material which have a largest dimension substantially in the range of 3 to 10 mm, such particles will in the manufacturing process of the diapers, to be described later on, become oriented mainly parallel to the plane of the diaper, and particles so oriented contribute to better permanence of shape and elasticity of the diaper. The said particles increase the rigidity in bending of the diaper, this being evident in accentuated manner when the particles are subjected to bending in the folds of the diaper. Moreover, said particles inhibit the straight passage of excretions through the diaper courses, because they are somewhat less permeable than the cellulose component proper, and thereby they direct the excretions to a wider area in the filling of the diaper. It is also an important advantage that newsprint is a substantially less expensive material than chemically manufactured cellulose.

In the diaper of the invention, the cellulose component and the newsprint component may be substantially uniformly distributed in the filler material of the diaper, but it may be advantageous in one instance to make stratified structures having at certain points mainly or exclusively paper component and at others, mainly or exclusively cellulose component material.

In the following is briefly described an apparatus and a procedure by which diapers according to the present invention are manufactured. The apparatus applied in the procedure comprises a conveyor belt 21 moving over return wheels. At the front end of the conveyor 20, 21 has been disposed, within the loop of the wire-like and permeable conveyor band 21, a suction box 25, from which a suction connector 27 leads to a vacuum pump 26. Above said suction box 25 is located a chamber 24, into which filling material is supplied through the pipe 23 from a hammer mill 22 or equivalent. Into the hammer mill is from the reel 29 conducted a cellulose web 29', consisting of fibrous cellulose. Into the same mill is conducted with higher speed, from the reel 28, a paper web 28', or a plurality of webs, so that an appropriate cellulose/paper component proportion is obtained. In the hammer mill 22 an appropriate sieve is employed so that an expedient size distribution of the paper component is obtained as the cellulose component is ground down to separate fibres.

Onto the conveyor belt 21 is supplied from the reel 31, a tissue paper web 31', upon which the filler material produced from the webs 28' and 29' is deposited with the aid of suction. Hereafter, the combination web thus obtained is cut into lengths and folded to have the form shown in Fig. 1, and the diaper blanks thus obtained are enclosed in bags formed from the non-woven web 30' coming from the reel 30, so that a diaper 10 as shown in Fig. 1 is produced. The device disclosed above is provided with previously known automatisation and cutting means, which are not necessary to be specified in detail in this connection.

**Claims**

1. Baby's diaper or equivalent consisting of a filler material containing comminuted newsprint or equivalent paper and free chemically manufactured cellulose fibres enclosed within a skin layer, characterized in that the paper is substantially in particles having a largest dimension of 3 to 10 mm, and the proportion of paper in the filler material is 40 to 60% by weight.

2. Diaper according to claim 1, characterized in that the paper has been made of pulp containing thermoground wood or other mechanical pulp with comparatively long fibres.

3. Diaper according to claim 1 or 2, characterized in that the paper and the free cellulose are substantially uniformly distributed in the filler material (14).

4. Diaper according to claim 1, 2 or 3, characterized in that paper and free cellulose are in a stratified arrangement in the filler material.

**Patentansprüche**

1. Babywindel oder dergleichen, aus einem innerhalb einer Außenhautschicht eingeschlossenen Füllmaterial mit zerkleinertem Zeitungsdruckpapier oder dergleichen und Faser-Zellulose, dadurch gekennzeichnet, daß das Papier hauptsächlich in Partikeln vorliegt, deren größte Abmessungen um 3 bis 10 mm liegen, und daß der Anteil des Papiers im Füllmaterial um 40 bis 60 Gew.-% beträgt.

2. Babywindel nach Anspruch 1, dadurch gekennzeichnet, daß das Papier aus einer Papierpulpe bzw. einem Papierstoff, der Warmschliff-Holzzellstoff enthält oder einer anderen mechanisch aufbereiteten Papierpulpe mit verhältnismäßig langen Fasern hergestellt ist.

3. Babywindel nach Anspruch 1 oder 2, dadurch gekennzeichnet, daß das Papier und die Zellulose im wesentlichen gleichmäßig im Füllmaterial (14) verteilt sind.

4. Babywindel nach Anspruch 1, 2 oder 3, dadurch gekennzeichnet, daß das Papier und die Zellulose im Füllmaterial in geschichteter Anordnung vorliegen.

**Revendications**

1. Couche pour bébé ou équivalent, consistant en un matériau de remplissage contenant du papier journal ou un papier équivalent réduit en menus fragments et des fibres de cellulose maigre fabriquée chimiquement, enfermé à l'intérieur d'une couche formant enveloppe, caractérisée en ce que le papier est sensiblement en particules dont les dimensions les plus importantes sont de 3 à 10 mm, et en ce que la proportion de papier dans le matériau de remplissage est de 40 à 60% en poids.

2. Couche selon la revendication 1, caractérisée en ce que le papier est réalisé à partir d'une pâte contenant du bois ayant subi un thermo-broyage ou toute autre pâte mécanique à fibres relativement longues.

3. Couche selon la revendication 2, caractérisée en ce que le papier et la cellulose maigre sont réparties sensiblement uniformément dans le matériau de remplissage (14).

4. Couche selon la revendication 1, 2 ou 3, caractérisée en ce que le papier et la cellulose maigre sont disposées en stratifications dans le matériau de remplissage.

FIG. 1

FIG. 2

FIG. 3